Europäisches Patentamt

European Patent Office

Office européen des brevets

(19)

(11) Publication number: **0 226 470**
**A2**

# (12) EUROPEAN PATENT APPLICATION

(21) Application number: **86309709.3**

(22) Date of filing: **12.12.86**

(51) Int. Cl.⁴: **G 01 N 33/543**
**// C12M1/40, G01N33/52**

(30) Priority: **13.12.85 GB 8530715**

(43) Date of publication of application:
**24.06.87 Bulletin 87/26**

(84) Designated Contracting States:
**AT BE CH DE ES FR GB GR IT LI NL SE**

(71) Applicant: **UNILEVER PLC**
**Unilever House Blackfriars P.O. Box 68**
**London EC4P 4BQ (GB)**

(84) Designated Contracting States: **GB**

(71) Applicant: **UNILEVER NV**
**Burgemeester s'Jacobplein 1 P.O. Box 760**
**NL-3000 DK Rotterdam (NL)**

(84) Designated Contracting States:
**BE CH DE ES FR GR IT LI NL SE AT**

(72) Inventor: **Bosley, John Anthony**
**10 Philip Way Higham Ferrers**
**Wellingborough Northamptonshire (GB)**

**Davis, Paul James**
**The Hawthorns Pavenham Road**
**Felmersham Bedfordshire MK 3 7EX (GB)**

**Shanks, Ian Alexander**
**Flintwood Cottage Channer Drive**
**Penn Bucks HP10 8AQ (GB)**

**Smith, Alan Martin**
**Littlebrook 92B Village Road**
**Bromham Bedford MK43 8HU (GB)**

(74) Representative: **Stancliffe, Terence Christopher et al**
**UNILEVER PLC Patents Division P.O. Box 68 Unilever House**
**London EC4P 4BQ (GB)**

(54) Materials and methods for microchemical testing.

(57) Apparatus for carrying out a microchemical test, comprising a solid substrate having a surface which carries a polymer hydrogel formed in situ thereon and covalently bonded thereto, for example with the gel in contact with a metal conferring microchemical analytical specificity on the apparatus, e.g. an immunological reactant or an electrode. For example, such a gel in a layer carried within a capillary-fill cell can be used for optical immunoassay.

EP 0 226 470 A2

Bundesdruckerei Berlin

**Description**

Materials and Methods for Microchemical Testing

This invention relates to materials and methods for microchemical testing, including specific binding assays including immunoassays (all of which specific binding assays are generally referred to herein as immunoassays).

Among relevant prior art methods of microchemical testing are large numbers of forms of immunoassay, including for example separation assays based on the use of solids carrying immunosorbent sensitisation, and a number of electrochemical test methods based on chemically sensitive transistors and other semiconductor devices, and other semiconductor devices, and chemically selective electrodes. Sometimes the use of immunological sensitisation has been combined with electrochemical methods of detection.

The prior art includes several techniques in which there is involved a coating, especially an envelopment, of glass beads or similar substrates, with a polymeric material, possibly including an immuno-reactant.

USP 4 478 946 (S.A. Inventions Devel.Corp.) and USP 4 352 884 (Kuraray) are both of this class, and both disclose cross-linking in-situ of polymer layers formed by coating a substrate with polymeric material.

GB 2 146 029 (Ramot University) is respresentative of techniques for making enzyme electrodes coated with acrylic polymers containing enzymes: these also are treated to cross-linking in situ after formation of a polymer coat.

USP 4 198 389 (Hoffmann-la Roche) discloses the use, in connection with alternating-currents and immuno-analysis methods based on electromobility, of various gels carried on solid supports, and containing immunologically active materials.

USP 4 360 358 (Sharma) discloses immunoassays using solid phase substrates coated with dried layers of organic polymer, containing immunologically active material.

USP 4 048 298 (Rohm & Haas) discloses insolubilisation or immobilisation of antibody to a solid phase by polymerisation with aldehyde or alkyl haloformate, or physical entrapment in an insoluble gel polymer or by covalent coupling or adsorption to water-insoluble polymer.

GB 2 046 448 (NRDC) discloses ion-selective electrodes having sensitised polymer membranes separating different liquid electrolytes.

USP 4 298 687 (Maes) discloses the use of sensitised polyacrylamide gels as solid phase in immunoassay.

USP 4 245 016 and 4 298 667 (GEC) describe coated electrodes and separators coated with acrylic copolymers.

USA 4 259 223 (Cal.Inst.Tech.) describes polymeric microspheres carrying surface activation and forming a film coating over porous glass particles.

USP 4 415 666 (Miles) describes electrochemical analysis multilayer membranes made by casting cellulose acetate compositions.

GB 2 119 162 (UKAEA) describes solid-state electrochemical cells containing solid electrolytes incorporating anionic acrylic polymer.

As seen from representative examples cited above, the prior art includes a large number of methods of providing immunological sensitisation of solid materials. Some have been used in commercial practice. In many cases, however, their manufacture is difficult to carry out while providing acceptable stability and reproducibility of the resulting materials.

We have found that with prior techniques it is difficult to produce uniform polymer or uniform polymer activation.

Accordingly, it is an aim of this invention to produce microchemical analytical apparatus comprising polymer layers, using techniques which can provide improved uniformity in the polymer layers.

A further aim of the invention is to facilitate production of polymer hydrogel layers with high standards of optical uniformity for the purpose of optical techniques of microchemical analysis including immunoassay.

It is one aim of this invention to provide immunologically sensitised solid materials and methods for their manufacture in a way that broadens the effective range of manufacturing techniques available to solve any given analytical problem, and to provide stable and storable compositions which can be conveniently and reproducibly manufactured.

Another aim of the invention is to provide new and useful composites for use in connexion with electrochemical testing.

According to an aspect of the invention we provide apparatus and compositions for carrying out immunological and other microchemical tests, comprising a solid substrate to which is fixed a gel, especially e.g. a hydrogel polymer, which either carries immunological sensitisation or is physically associated with some other material conferring microchemical analytical specificity, e.g. an electroactive membrane and/or chemically selective electrode.

According to the invention we also provide apparatus for carrying out a microchemical test, comprising a solid substrate having a surface which carries a polymer hydrogel formed in situ thereon and covalently bonded thereto. In such apparatus, the gel can be in contact with a material conferring microchemical analytical specificity on said apparatus. For example, the gel can carry an immunological reagent which is a ligand or a binding agent therefor. In certain embodiments, incorporating crosslinked gel, said ligand or binding agent can be a macromolecule occluded within the gel. Alternatively, the ligand or binding agent can be covalently bonded to said gel.

The substrate used in this invention can be for example a siliceous substrate surface-coated with an organic trialkoxy silane derivative which carries an organic functional group conjugated with a mer of the polymer hydrogel.

The polymer hydrogel can be for example a cross-linked acrylic polymer hydrogel covalently linked to said surface by polymerisation in situ via residues of said organic trialkoxysilane carrying residues of amino-alkyl or epoxyalkyl or acryloyl groups, and optionally carrying organic functional groups derived from comonomers which are copolymerisable with acrylic monomers and which comprise N-hydroxysuccinimidyl, hydroxyethyl, carboxyl or aldehyde groups.

In certain embodiments, the solid substrate can be a plate carrying a layer of said gel thereon, e.g. on an internal surface of a capillary-fill cell formed by two closely-spaced parallel plates. In certain other embodiments, said surface of said solid substrate carries material in contact with said gel and forming an electrochemical electrode. In yet other embodiments said gel can carry at least one enzyme and at least one ligand or receptor therefor, optionally with a chromogenic or fluorogenic enzyme substrate occluded within said gel.

Also provided by the invention is a process for producing a gel carried on a surface of a solid substrate, which comprises:

(a) activating the surface of the substrate to provide covalently-attached functional groups which are capable of reacting with a polymerising monomer, and

(b) polymerising a polymerisable monomer solution in a layer in contact with said activated surface, thereby to form in situ a layer of polymer gel covalently linked to said surface. In this process, the monomer solution can be an aqueous preparation of polymerisable acrylic monomer, and for example a ligand or specific ligand binding agent can be present in the aqueous monomer preparation.

The solid substrate can be part of a test apparatus of any shape or form, ranging from the simple, e.g. a bead, or end of a handling-piece, or strip of substrate material, to more complex apparatus, such as a cell or wall or cuvette of apparatus to contain assay liquid, or an internal surface of sampler apparatus as described in EP 0 164 180 = WO 85/04255, or of capillary fill cell apparatus as described in European Patent Application No. 85304169.7 (EP 0 171 148), which is incorporated herein by reference. For example, the solid substrate can be a glass or other siliceous or ceramic surface, as of a glass-walled vessel or a silica layer covering a solid material of another composition. Alternatively, the solid substrate can be of plastics material.

In the case of embodiments of the invention comprising electrodes, especially chemically selective electrodes and/or electroactive membrane, other features of construction can be for example as described in European Patent Application No. 85304170.5 (EP 0 170 375) or EP 0 186 286, both of which are incorporated herein by reference.

In particular embodiments, such a glass or siliceous layer can be a very thin layer (e.g. a few microns thick) which has been deposited or grown on a composite structure incorporating an electrode and/or a semiconductor device such as for example a field-effect transistor, optionally with an electroactive overlayer to impart electrochemical selectivity.

The preferred material for bonding the gel to the solid surface is a superficial layer on the solid surface which has been derived by applying to the solid surface a substituted trialkoxysilane carrying a substituent which can either take part directly in gel formation, e.g. as a polymerisable comonomer group, or else take part indirectly, e.g., after its later substitution with such a polymerisable comonomer group.

The presently preferred compound for this purpose is methacryloyloxypropyltrimethoxysilane (MOPS).

Alternatively, another substituted silane can be used, with a functional group capable of substitution by a polymerisable comonomer. Gamma-aminopropyl trialkoxysilanes and glycidyloxypropyltrialkoxysilanes are suitable examples, and they can be substituted with for example acryloyl halide or acrylamide after application to the solid surface.

Silane derivatives can be applied to chemically clean glassy or siliceous surfaces by contact as solutions in an appropriate solvent, for example (dry) toluene at about 70°C for a few hours, or aqueous solution at pH about 3.5 to 3.6, e.g. at about 20°C for a few hours. These examples of conditions are mentioned for illustration and not limitation. The gels to be formed on the solid surfaces are preferably gels based on acrylic monomer units, especially acrylamide and hydroxyethyl methacrylate, including a proportion of cross-linker. Any monomers that can copolymerise with such acrylic monomers can be a usable comonomer unit for this purpose. Preferred comonomers are acrylamide as the base and methylenebisacrylamide as crosslinker. The proportion of cross-linking is chosen to give a desired degree of porosity to the gels, and may be very low (e.g. of the order of about 0.0015% to 0.025% by mole), if the desired degree of porosity is to be such as will allow the entry by diffusion of large molecules such as proteins; or somewhat higher (e.g. of the order of about 0.25% by mole) if the gel should be such as to exclude large proteins such as antibodies.

The gels preferably are formed with substituted functional groups derived for example from functional comonomer derivatives of acrylic acid or acrylamide (including substituted acrylic acids and acrylamides), e.g. from N-hydroxysuccinimidyl acrylate (NHSA) at up to a few tens of mole %, e.g., 10-20 mole %, or for example from hydroxyethyl methacrylate (HEMA) in similar proportions by mole.

Some useful functional groups can be such as to react directly with proteins, e.g. acrolein as a comonomer which yields aldehyde groups. Others may be such as to require activation after the polymer has formed before they can react with protein, e.g. HEMA or acrylic acid, which provide hydroxy(alkyl) and carboxyl groups respectively.

The use of NHSA to functionalise polyacrylamide gels is known per se and is for example described with useful details by R.L. Schnaar and Y.C. Lee in Biochemistry (1975), 14(7) pp 1535-1541.

Other derivatisation procedures known per se can be easily adapted for application to the gels discussed herein.

Among alternative substrates on which gels can be fixed to make test materials in accordance with this invention are the following:- an oxidised metal wire or plate, e.g., of oxidised titanium, or oxidised or silica-coated silver, (e.g. a 50-500 nm silver mirror plated on a glass or plastics prism or fibre and covered with a (approx.) 1 nm coating of silica), treated to apply a layer of silane-derived material as described above.

Also usable are plastics substrates. In the case of plastics substrates, the invention includes the use for example of plastics surfaces derivatised to include covalently bonded acryloyl or glycidyl groups, which can be achieved either directly or by the use of intermediately derivatised plastics surfaces (known per se) comprising covalently bonded amino, carboxyl or hydroxyl groups which are then reacted with a functional reagent capable of copolymerising with acrylic monomer, e.g. acryloyl or methacryloyl chloride.

The invention is further illustrated by the following examples.

Example 1

A capillary-fill cell can be made in the following way to incorporate a thin layer of gel fixed to a transparent optical flat surface of the cell, the gel carrying covalently bound antibody. The cell can be used to carry out an immunoassay as described below.

The bonding tracks can be applied to the glass either before or after the gel formation, preferably afterwards. The gel-forming material can be applied either continuously or patchwise or in spots or other discrete areas over the surface on which the gel is to form. The bonding tracks may then be applied either over the gel layer or over a part of the surface not carrying gel.

(a) A glass microscope slide (with one end optionally polished to give an optically flat perpendicular end) is cleaned successively with laboratory detergent, hot ammonia/hydrogen peroxide solution, and hot hydrochloric acid/hydrogen peroxide solution. The clean slide is contacted with 2% v/v methacryloyloxypropyltrimethoxysilane (MOPS) (Aldrich Chemical Co.), used as a solution in aqueous medium (4 ml commercially available material dissolved in 1 litre of water containing a small quantity of acetic acid to give a final pH approx. 3.5). This solution is stirred for about 15 minutes until clear. The clean slide is contacted with the solution and shaken for about 90 minutes at room temperature (about 22° C), and the slide is then washed with distilled water and air-dried at room temperature.

(b) The MOPS-treated glass slide is next contacted with an approx. 15-micron-thick layer of activated comonomer solution based on acrylamide with a very low amount of crosslinking methylenebisacrylamide (up to about 0.025% by mole) so that the resulting gel will have a large pore size into which protein molecules can diffuse. Also included in the comonomer mixture is 10%-20% (by mole) N-hydroxysuccinimidylacrylate as activated monomer which can later be substituted by functional groups such as antibody protein molecules, antigens, or haptens. In alternative and sometimes preferred embodiments, the proportion of crosslinker can be much lower, e.g. 0.0015% by mole.

The activator for polymerising the comonomer mixture is a conventional initiator mixture, e.g. 2,2'-azobis (2-amidinopropane) dihydrochloride, used at a concentration of about 0.12% based on total monomers. A preferred alternative is Irgacure 184 (Trade Mark), (UV photoinitiator), i.e. 1-hydroxycyclohexylphenyl ketone. Initiation is triggered by light or heat. The monomer solutions are all made up in purified water where appropriate, though aqueous buffer can be used if desired, and where NHS acrylate is used, it can be dissolved in acetone, and mixed with aqueous acrylamide to give a final proportion of 5:3 water:acetone, with the initiator dissolved first in whichever ingredient (e.g. acetone) provides greater solubility.

The thickness of the film (in this example, of the order of, e.g. 10 micron thick) can be controlled by adjusting monomer aliquot volumes at a fixed rate of surface area coverage.

The smoothness of the gel layer can (if desired) be further assured by clamping a flat solid surface in the desired spaced relationship to the microscope slide on which the gel layer is to be formed.

It is preferred to make such a top plate specially hydrophobic by coating it with dichlorodimethylsilane or equivalent anti-adhesive material.

(c) After formation of the gel, the gel is reacted for about 16 hours or overnight with aqueous antibody solution, for example, as here, anti-gentamicin antibody, at pH about 6-9, and about 2 mg/ml protein concentration, to allow antibody to diffuse into the gel and become covalently coupled to the gel by substitution of the N-hydroxy-succinimidyl groups. The quantity of active antibody introduced can be measured by conventional means, e.g. by binding of labelled (e.g. radioiodine labelled) antiglobulin.

The slide, so provided with antibody-bearing adherent silane-bonded gel layer, is then made up into a capillary-fill cell using the technique of European Patent Application No. 85304169.7 (EP 0 171 148).

(d) Immunoassay is carried out in the cell by introducing a mixture of (i) sample liquid containing an unknown quantity of gentamicin or a calibration standard, and (ii) fluorescent gentamicin (reaction product of gentamicin with FITC) in a standardised quantity suitably related to the quantity of antibody attached to the gel within the cell for the purposes of competitive immunoassay.

After a suitable reaction period, the fluorescence bound to the gel is measured by a modification of the instrument disclosed in European Patent Application No. 85304172.1 (EP 0 170 376) which is incorporated herein by reference, so that the output signal is taken to be proportional to the light which emerges from the optically flat end of the microscope slide at angles within a marginal range slightly closer to the axis than the innermost angular limit on each side at which most light derived from the bulk of the liquid emerges from the slide. In one example, based on a glass substrate (refractive index about 1.52), aqueous reaction liquid (refractive index about 1.33), and a gel with refractive index about 1.38 when hydrated, the suitable range of angles of exit off the long axis of the slide was found to be within about 39.5° to 47.4° on either side of the axis.

## Example 2

According to this Example, gel layers of about 30-500 micron in thickness are formed on the surface of one end each of glass slides prepared and treated with MOPS as in Example 1. The gel-forming material is equivalent to that used in Example 1 of European specification No. 85306572.0 (EP 0 178 790), i.e., it includes biotinyl-HEMA comonomer and glucose oxidase for occlusion in the gel. The resultant gel bodies, fixed on the ends of the glass slides as solid substrates, can be used as a convenient material for carrying out assays in ways corresponding to those mentioned and described in European specification No. 85306572.0, (EP 0 178 790), which is incorporated herein by reference.

## Example 3

A silica-coated glass slide cut from Permabloc float glass (ex Pilkington) and cleaned with laboratory detergent is treated with MOPS in the manner decribed in Example 1.

The MOPS-treated glass slide is then contacted with an approx. 6 micron thick layer of an active comonomer solution based on 1-vinyl-2-pyrrolidone with a low amount of difunctional ethylene glycol dimethacrylate (for example, 0.5% by mole). The resulting gel has a large pore size into which protein molecular can diffuse. Also included in the comonomer mixture is 10-20 mole % acrolein as active monomer which can later be substituted by functional groups such as antibody protein molecules, antigens, or haptens.

The activator polymerising the comonomer mixture is a conventional photoinitiator, e.g. 1-hydroxycyclohexylphenyl ketone, used at a concentration of approx. 0.35% based on total monomers. Initiation is triggered by long wavelength u.v. light. The monomer solutions may be made up in a solvent, e.g. 1-methyl-2-pyrrolidone, or in purified water, or aqueous buffer.

## Example 4

In Example 1, the step involving the binding of antibody to active sites within a gel after polymerisation can be made unnecessary by polymerising an aqueous monomer solution that contain antibodies.

For example, a MOPS-treated glass slide described in Example 1 or 3 is contacted with an approx. 6 micron thick layer of an active comonomer solution based on acrylamide. Also included in the comonomer mixture is 10-20 mole % as active monomer and a very small amount of the cross-linking agent N,N'-methylenebisacrylamide (approx. 0.0015 mole %).

The monomers are made up in aqueous buffer solution containing for example rabbit anti-human IgG. The initiation system consists of Quantacure QTX (Tradename, ex Ward Blenkinsop & Co. Ltd) and dimethylaminoethanol. (Quantacure QTX comprises 2-hydroxy-3-(3,4-dimethyl-9-oxo-9H-thioxanthen-2-yloxy)-N,N,N-trimethyl-1-propylammonium chloride). The polymerisation of the comonomer solution is triggered by exposure to visible light from a tungsten lamp. After polymerisation, the gels are washed in phosphate-buffered saline.

## Example 5

In addition to the use of the active monomers described in Examples 1, 3 and 4, it is also possible to use monomers that may be activated in a separate step following polymerisation.

For example, a MOPS-treated glass slide described in Examples 1 or 3 is contacted with an approx. 6 micron thick layer of an aqueous comonomer solution consisting of acrylamide (49.5 mole %), sodium acrylate (49.5 mole %) and N,N'-methylenebisacrylamide (1 mole %).

The activator polymerising the comonomer mixture is a conventional photoinitiator, e.g. 1-hydroxycyclohexylphenyl ketone, used at a concentration of approx. 0.16 % based on total monomers. Initiation is triggered by long wavelength u.v. light.

Following polymerisation, the carboxyl groups contained within the polymer matrix may be activated by treatment with, for example, an aqueous solution Woodward's Reagent K (N-ethyl-5 phenylisoxazolium-3'-sulphonate). The activated copolymer may then be reacted with functional groups such as antibody protein molecules. antigens, or haptens.

An embodiment of the invention is illustrated for example by the accompanying Figures 1-3 and associated description.

Figure 1 shows a diagrammatic section through a disposable capillary cell device incorporating a gel layer formed according to one embodiment of the invention.

Figure 2 shows a diagrammatic plan of the cell device of Figure 1, and includes a line I-I to show the line of the section of Figure 1.

Figure 3 shows in diagrammatic fragmentary plan an intermediate stage in the manufacture of a plurality of devices as of Figures 1-2.

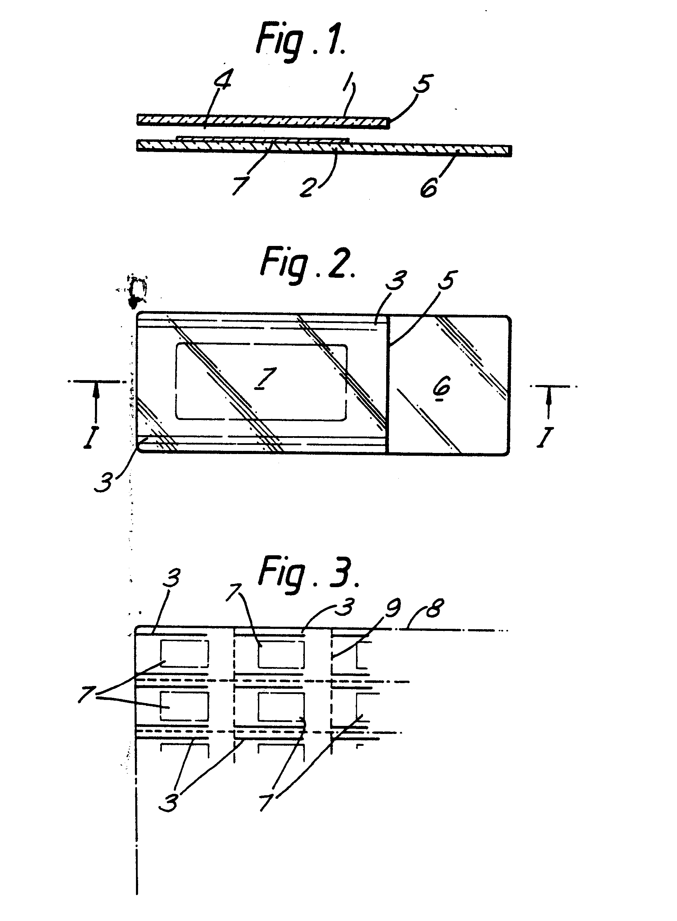

Figures 1-2 show a capillary cell device of a size to be handled easily, e.g. about 3 cm × 1.5 cm. The device comprises an upper plate 1 and lower transparent plate 2 (about 1 mm thick), e.g. of glass, silica or plastics, fixed together in parallel opposed and spaced relation. less than 1 mm apart, e.g. 0.1 mm apart, by bonding tracks 3 of suitable (e.g. epoxy) adhesive to form a capillary cell cavity 4, open at both ends, which communicates with the outside through a first discontinuity in the binding 3 arranged to form a cell aperture at side 5 of plate 1. Another discontinuity is present at the other end of bonding 3, to leave another aperture, to allow exit of air when a sample liquid is loaded into the cell. Plate 2 is larger than plate 1 and has a portion 6 extending away from the aperture. Portion 6 of plate 2 acts as a platform or threshold or lip onto which a drop of sample liquid can be applied, so that this liquid can be made to fill the capillary cell cavity 4 by capillary flow. Cavity 4 attracts and contains a definite and adequately reproducible volume of liquid when loaded in this way.

Attached to the inner surface of the capillary cell is a layer 7 including gel and other specific material relevant to the test procedure in which the capillary cell is to be used. In the example shown in the drawings the layer 7 is a patch of hydrogel including antibody, carrier on plate 2. There can be more than one such layer, e.g. a layer on plate 1 as well as plate 2, or a superimposition and/or side-by-side plurality of layers on either plate. Fir similar or other purposes, the layer 7 or other layer(s)m lining the internal surface(s) of the capillary cell can include an electically conductive layer or layers, as described in European Application No. 85304170.5 (EP 0 170 375), incorporated herein by reference, and in such a case conductive external connections can be provided by means of conductive tracks or connectors from the interior of the cell, if desired, passing between bonding layer 3 and the surface of the plates. These can for example be made in a manner known per se and used in the conventional surface fabrication of conductive tracks as often employed in the manufacture of semiconductors and liquid crystal displays.

When the cell is intended for making optical measurements, either plate 1 or plate 2 or both should be transparent or translucent.

The section shown as Figure 1 presents plates 1 and 2 spaced apart because the line of section does not extend through the bonding tracks 3.

The fabrication of a plurality of cells such as that of Figures 1-2 is illustrated by Figure 3, a fragmentary plan diagram showing an intermediate stage in the manufacture of such cells. A large plate 8 of glass or other material to make plates 2 is cleaned and coated in an appropriate way with patches of material 7 of the kinds described above as well as tracks of bondable adhesive 3. A second plate, not shown, is then adpressed to plate 8, optionally after forming on it bonding tracks corresponding to track 3, and optionally after forming patches or tracks of any other desired material, and the adhesive is cured. Then the assembly is broken or cut along lines shown as dotted lines 9 in Figure 3, and corresponding lines in the upper plate (not necessarily in registration with lines 9, though). The result is to give cells like the cells shown in Figures 1-2.

Further modifications and variations of devices according to the invention can be for example as discussed in European Patent Application No. 85304169.7 (EP 0 171 148).

The composite material provided by this invention, comprising gels carried on for example macro-solid phase materials, e.g. slides, beadlet, strip, tube, rod, peg or stick material, can be stored and presented to the user either in wet form or in dry rehydratable form. In either case, chemical/biological preservative can be included in the gel material, as well as any desired test reagents. Auxiliary test reagents can additionally/alternatively be present in or on any desired other part of the solid phase that carries the gel, e.g. as a film or printed spot or patch or impregnated fibrous or membranous material, e.g., filter material of cellulosic or synthetic polymer material. All or part of the materials may be stored and presented in hermetically sealed form, e.g., within a sealed foil wrapping or closure or within an appropriately sealed or closed container within which the test reactions are to be carried out.

The skilled reader will understand that the features mentioned and cited in this description and the claims, together with those of each of the cited European patent applications, are disclosed and may be used in any combinations, subcombinations and permutations.

**Claims**

1. Apparatus for carrying out a microchemical test, comprising a solid substrate having a surface which carries a polymer hydrogel formed in situ thereon and covalently bonded thereto.

2. Apparatus according to claim 1, wherein said gel is in contact with a material conferring microchemical analytical specificity on said apparatus.

3. Apparatus according to claim 2, wherein said gel carries an immunological reagent which is a ligand or a binding agent therefor.

4. Apparatus according to claim 2, wherein said gel is crosslinked and said ligand or binding agent is a macromolecule occluded within said gel.

5. Apparatus according to claim 3, wherein said ligand or binding agent is covalently bonded to said gel.

6. Apparatus according to claim 1, comprising a siliceous substrate surface-coated with an organic trialkoxy silane derivative which carries an organic functional group conjugated with a mer of the polymer hydrogel.

7. Apparatus according to claim 6, wherein the polymer hydrogel is a cross-linked acrylic polymer hydrogel covalently linked to said surface by polymerisation in situ via residues of said organic trialkoxysilane carrying residues of amino-alkyl or epoxyalkyl or acryloyl groups and optionally carrying organic functional groups derived from comonomers which are copolymerisable with acrylic monomers and which comprise N- hydroxysuccinimidyl, hydroxyethyl, carboxyl or aldehyde groups.

8. Apparatus according to claim 1, wherein said solid substrate is a plate carrying a layer of said gel thereon.

9. Apparatus according to claim 8, wherein said gel is carried on an internal surface of a capillary-fill cell formed by two closely-spaced parallel plates.

10. Apparatus according to claim 1, wherein said surface of said solid substrate carries material in contact with said gel and forming an electrochemical electrode.

11. Apparatus according to claim 1, wherein said gel carries at least one enzyme and at least one ligand or receptor therefor, optionally with a chromogenic or fluorogenic enzyme substrate occluded within said gel.

12. A process for producing a gel carried on a surface of a solid substrate, which comprises:

    (a) activating the surface of the substrate to provide covalently-attached functional groups which are capable of reacting with a polymerising monomer, and

    (b) poymerising a polymerisable monomer solution in a layer in contact with said activated surface, thereby to form in situ a layer of polymer gel covalently linked to said surface.

13. A process according to claim 12, wherein the monomer solution is an aqueous preparation of polymerisable acrylic monomer.

14. A process according to claim 13, wherein a ligand or specific ligand binding agent is present in the aqueous monomer preparation.

0226470

Fig.1.

Fig.2.

Fig.3.